# EUROPÄISCHE PATENTANMELDUNG

(11) **EP 2 886 162 A1**
(43) Veröffentlichungstag der Anmeldung: **24.06.2015**
(21) Anmeldenummer: 14159846.6
(22) Anmeldetag: 14.03.2014
(51) Int. Cl.: A61Q 5/12, A61K 8/92, A61K 8/81, A61K 8/41, A61K 8/49, A61K 8/37, A61K 8/64, A61K 8/31

(54) **Haarpflegemittel mit ausgewählten Homo- oder Copolymeren auf Basis von Pflanzenölen**

(30) Priorität: 18.12.2013 DE 102013226540
(71) Anmelder: Henkel AG & Co. KGaA, 40589 Düsseldorf (DE)
(72) Erfinder: Mette, Manuela, 23923 Kleinfeld (DE); Hippe, Thomas, 25482 Appen (DE)

(57) **Zusammenfassung**

Die Erfindung betrifft Haarbehandlungsmittel enthaltend in einem geeigneten kosmetischen Träger, jeweils bezogen auf sein Gesamtgewicht,
a) mindestens ein Homo- oder Copolymer enthaltend mindestens ein Monomer aus der Gruppe der Pflanzenöle in einer Gesamtmenge von 0,01 bis 10 Gew.-%,,
b) mindestens eine quartäre Ammoniumverbindung in einer Gesamtmenge von 0,01 bis 8 Gew.-%,
c) mindestens ein Esteröl in einer Gesamtmenge von 0,01 bis 10 Gew.-%
sowie die haarkosmetische Verwendung dieser Mittel und Verfahren unter Einsatz dieser Mittel.

## Beschreibung

Die vorliegende Erfindung betrifft ein kosmetisches Haarbehandlungsmittel enthaltend ausgewählte Homo- oder Copolymere, quarternären Ammoniumverbindungen und Esteröl als Pflegestoffe, ein Verfahren unter Verwendung der Mittel sowie die Verwendung der Mittel zur Behandlung von Haaren, insbesondere zur Pflege der Haaren, zur Verbesserung der Nass- und Trockenkämmbarkeit und zur Verbesserung der Widerstandsfähigkeit der Haaroberfläche gegenüber physikalischen Schädigungen, wie z.B. Kämmen und Föhnen, und ganz besonders bevorzugt zur Verbesserung des Anti-Spliss Effekt bzw. zur Verbesserung des Anti-Haarbruches. Vielfach wiederholte Haarbehandlungen, wie beispielsweise oxidative oder reduktive Haarbehandlungen, aber auch die allzu häufige Reinigung der Haare, die Einwirkung von Fönhitze sowie Umwelteinflüsse und/oder mechanische Einwirkungen, können Haare in ihrer Struktur schädigen. Die Folgen der Schädigungen sind oftmals Haarbruch, Spliss sowie glanzlose, stumpfe und/oder fliegende Haare.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quartären Ammoniumsalzen oder speziellen Polymeren, behandelt. Durch diese Behandlung werden je nach Formulierung die Kämmbarkeit, der Halt und die Fülle der Haare verbessert und die Splissrate verringert.

Es hat sich jedoch gezeigt, dass die Qualität und/oder Wirksamkeit der Nachbehandlungsprodukte je nach Wahl der Pflegekomponenten stark variieren kann. So konnte beispielsweise beobachtet werden, dass die Abscheidung von Pflegekomponenten auf den Haaren -je nach Kombination der Wirkstoffe in dem Haarpflegemittel - nicht immer optimal ist, wodurch die Pflegeleistung der Mittel unbefriedigend wird.

Weiterhin ist es nicht möglich, beliebige Mengen haarpflegender Komponenten - insbesondere wasserunlöslicher Haarpflegekomponenten - in die Nachbehandlungsmittel einzuarbeiten. So treten ab einem gewissen Gehalt an Ölen oftmals Separationserscheinungen auf, und die Hitzeund/oder Kältestabilität der Produkte ist ebenfalls oftmals unbefriedigend.

Es besteht daher weiterhin ein Bedarf nach Wirkstoffen bzw. Wirkstoffkombinationen für lagerstabile Haarbehandlungsmittel mit guten pflegenden Eigenschaften, die Haarschädigungen verhindern oder verringern, und möglichst auf Naturstoffen basiert sind. Insbesondere wird die Herstellung von einem Pflegekomplex angestrebt, der idealerweise auch in Verbindung mit Oxidationsmitteln und tensidischen Mitteln eingesetzt werden kann.

Es wurde nun überraschenderweise gefunden, dass die Aufgabe in hervorragendem Maße durch ein Haarbehandlungsmittel, welches einen Wirkstoffkomplex enthaltend als wesentliche Inhaltsstoffe mindestens ein ausgewähltes Homo- oder Copolymer, mindestens eine quarternären Ammoniumverbindung und mindestens ein Esteröl enthält, gelöst wird.

Haarbehandlungsmittel, welche diesen Wirkstoffkomplex enthalten, führen zur Pflege der Haaren, zur Verbesserung der Nass- und Trockenkämmbarkeit und zur Verbesserung der Widerstandsfähigkeit der Haaroberfläche gegenüber physikalischen Schädigungen, wie z.B. Kämmen und Föhnen, und ganz besonders bevorzugt zur Verbesserung des Anti-Spliss Effekt und/oder zur Verbesserung des Anti-Haarbruches.

Ein erster Gegenstand der vorliegenden Erfindung ist daher ein Haarbehandlungsmittel enthaltend in einem geeigneten kosmetischen Träger, jeweils bezogen auf sein Gesamtgewicht,
a) mindestens ein Homo- oder Copolymer enthaltend mindestens ein Monomer aus der Gruppe der Pflanzenöle in einer Gesamtmenge von 0,01 bis 10 Gew.-%,
b) mindestens eine quartäre Ammoniumverbindung in einer Gesamtmenge von 0,01 bis 8 Gew.-%,
c) mindestens ein Esteröl in einer Gesamtmenge von 0,01 bis 10 Gew.-%. Haarbehandlungsmittel im Sinne der vorliegenden Erfindung sind beispielsweise Haarshampoos,

Haarkonditionierer, konditionierende Shampoos, Haarspülungen, Haarkuren, Haarpackungen, Haar-Tonics, Haarfärbeshampoos oder deren Kombinationen. Insbesondere werden das Haar konditionierende Zusammensetzungen wie Haarspülungen, Haarkuren, Haarpackungen, Haaröle und- lotionen sowohl als leave-on, also als auf dem Haar bis zur nächsten Haarwäsche verbleibende Produkte, als auch als rinse-off, also wenige Sekunden bis wenige Stunden nach der Anwendung wieder auszuspülende Produkte, unter den erfindungsgemäßen Haarbehandlungsmitteln verstanden.

Unter Kämmbarkeit versteht sich erfindungsgemäß sowohl die Kämmbarkeit der nassen Faser, als auch die Kämmbarkeit der trockenen Faser.

Weiterhin zeichnen sich die erfindungsgemäßen Zusammensetzungen enthaltend den erfindungsgemäßen Wirkstoffkomplex durch einen deutlich verbesserten Zustand der keratinischen Fasern in Bezug auf den Feuchtehaushalt der keratinischen Fasern aus. Weiterhin führt der erfindungsgemäße Wirkstoffkomplex zu einem deutlichen Schutz der keratinischen Fasern vor Hitzeeinwirkungen, beispielsweise beim Föhnen keratinischer Fasern. Der Schutz der Oberfläche von keratinischen Fasern vor Hitzeeinwirkung ist insbesondere bei der Verwendung von Glätteisen oder Haartrocknern von großer Bedeutung. Schließlich wurde überraschender Weise festgestellt, dass die erfindungsgemäßen Zusammensetzungen zu einer deutlich verzögerten Wiederanschmutzung der keratinischen Fasern führen. Außerdem wird die Bildung von Schuppen auf der Kopfhaut deutlich verzögert.

Der erste obligatorische Bestandteil des erfindungsgemäßen Wirkstoffkomplexes sind Homo- oder Copolymere, enthaltend mindestens ein Monomer aus der Gruppe der Pflanzenöle.

Als Inbegriff für nachhaltige Synthesen kann die Nutzung von Pflanzenölen zur Herstellung neuartiger Polymere angesehen werden. Heutzutage werden, mit steigender Tendenz, Pflanzenöle oder ihre Derivate in der kosmetischen Industrie eingesetzt, um Materialien mit hervorragenden Anwendungseigenschaften zu erhalten und auch um unabhängiger von petrochemischen Erzeugnissen zu werden. Neue Synthesewege und die Optimierung herkömmlicher Verfahren liefern ein breites Spektrum an pflanzenölenbasierten Polymeren mit unterschiedlichsten Eigenschaften. Chemisch gesehen handelt es sich bei Pflanzenölen um Triglyceride, d.h. Triester des dreiwertigen Alkohols Glycerins mit unterschiedlich langkettigen Fettsäuren.

Für die kosmetische Nutzung ist vor allem die Zusammensetzung der Fettsäuren von großer Bedeutung. Diese variiert je nach Art der Pflanze, der Ernte, der Jahreszeit, den Anbau- sowie den Witterungsbedingungen, insbesondere nach Art der Pflanze.

Unter der Bezeichnung Fettsäure versteht man Substanzen, die eine oder mehrere Mehrfachbindungen in der Kohlenstoffkette aufweisen, welche sich im Gegensatz zu Einfachbindungen chemisch relativ einfach und vielseitig modifizieren lassen.

Zu den bekanntesten ungesättigten Fettsäuren zählen die Ölsäure, Linolsäure und Linolensäure, die in unterschiedlichen Mengen in Soja-, Raps-, Sonnenblumen- und Leinsamenöl vorkommen. Erfindungsgemäß geeignete Pflanzenöle sind beispielweise Rapsöl(auch Rüböl, Rübsenöl, Kolzaöl oder Kohlsaatöl genannt), Tungöl (auch chinesisches Holzöl genannt), Aprikosenkernöl, Arganöl, Avocadoöl, Babassuöl, Baumwollsamenöl, Distelöl, Erdnussöl, Hanföl, Haselnussöl, Kokosöl, Kürbiskernöl, Leinöl, Macadamianussöl, Maiskeimöl, Mandelöl, Mangobutter, Mohnöl, Nachtkerzenöl, Olivenöl, Palmöl, Palmkernöl, Rizinusöl, Sanddornfruchtfleischöl, Sheabutter, Sesamöl, Sojaöl, Sonnenblumenöl, Traubenkernöl, Walnussöl, Weizenkeimöl oder Wildrosenöl. Erfindungsgemäß bevorzugte Pflanzenöle sind beispielweise Rapsöl, Tungöl, Avocadoöl, Haselnussöl, Kokosöl, Macadamianussöl, Mandelöl, Mohnöl, Olivenöl, Palmöl, Palmkernöl, Rizinusöl, Sheabutter, Sesamöl, Sojaöl, Sonnenblumenöl, Traubenkernöl, Walnussöl, Weizenkeimöl oder Wildrosenöl.

Erfindungsgemäß ganz bevorzugte Pflanzenöle sind beispielweise Rapsöl, Tungöl, Avocadoöl, Kokosöl, Palmöl, Palmkernöl, Sojaöl, Traubenkernöl oder Walnussöl, und insbesondere Rapsöl oder Tungöl.

Zur Gewinnung von Rapsöl werden vor allem die Samen von Raps (Brassica napus) oder auch die Samen des nah verwandten Rübsens, wie z.B. Wild-Rübsen (Brassica rapa subsp. Campestris) oder Öl-Rübsen (Brassica rapa subsp. oleifera), genutzt. Erfindungsgemäß besonders bevorzugt sind Homo- oder Copolymere a), die mindestens ein Monomer aus Rapsöl, insbesondere aus Brassica Campestris öl, enthalten.

Tungöl wird in Südasien durch Auspressen der Samen baumförmiger Arten der Gattungen Vernicia und Aleurites gewonnen. In China wird meist der Tung- oder Abrasinbaum (Vernicia montana) und der Tungölbaum (Vernicia fordii, auch Aleurites fordii genannt), in Japan Aleurites cordata genutzt. Erfindungsgemäß besonders bevorzugt sind Homo- oder Copolymere a), die mindestens ein Monomer aus Tungöl, insbesondere aus Aleurites fordii öl, enthalten.

Von anderen Pflanzenölen unterscheidet sich Tungöl durch seine besondere chemische Zusammensetzung: Es besteht zu einem Großteil aus dem Glyzerid einer einzigen, dreifach ungesättigten Fettsäure, der α-Elaeostearinsäure. Weiterhin sind Ölsäurereste, Palmitinsäurereste und Stearinsäurereste - jeweils als Glycerinester gebunden - enthalten. Erfindungsgemäß besonders bevorzugt sind die Homo- oder Copolymere a), die mindestens ein Monomer aus der Gruppe der α-Elaeostearinsäureester, vorzugsweise aus der α-Elaeostearinsäureglyzerid, enthalten.

Ein erfindungsgemäß ganz bevorzugtes Haarbehandlungsmittel ist dadurch gekennzeichnet, dass die Polymer a) mindestens ein Monomer aus Rapsöl oder Tungöl, vorzugsweise aus Brassica Campestris Öl oder Aleurites Fordii Öl enthalten.

Ein erfindungsgemäß ganz bevorzugtes Haarbehandlungsmittel ist dadurch gekennzeichnet, dass es als Polymer a) mindestens ein unter der INCI-Bezeichnung: Brassica Campestris/Aleurites Fordii Oil Copolymer bekanntes Polymer enthält.

Ein weiteres erfindungsgemäß bevorzugtes Haarbehandlungsmittel ist dadurch gekennzeichnet, dass es als Polymer a) ausschließlich ein unter der INCI-Bezeichnung: Brassica Campestris/Aleurites Fordii Oil Copolymer bekanntes Polymer enthält.

Die Polymer(e) a) sind in den erfindungsgemäßen Haarbehandlungsmitteln in Gewichtanteil am Gesamtgewicht des Mittels zwischen 0,1 und 10 Gew.-%, vorzugsweise zwischen 0,2 und 5 Gew.- %, und insbesondere zwischen 0,4 und 3 Gew.-% erhalten.

Der zweite obligatorische Bestandteil des erfindungsgemäßen Haarbehandlungsmittels sind quartäre Ammoniumverbindungen. Quartäre Ammoniumverbinungen sind prinzipiell monomere kationische oder amphotere Ammoniumverbindungen, monomere Amine, Aminoamide, polymere kationische Ammoniumverbindungen sowie polymere amphotere Ammoniumverbindungen. Es existieren drei verschiedene Typen von quartären Ammoniumverbindungen und deren Untergruppen, nämlich:
- lineare Alkylammonium-Verbindungen
   - Alkyltrimethylammoniumsalze,
   - Dialkyldimethylammoniumsalze
   - Benzalkoniumsalze,
   - Esterquats
   - Ethoxylierte QAV
- Imidazolium-Verbindungen
- Pyridinium-Verbindungen

Aus dieser Vielzahl an möglichen quartären Ammoniumverbindungen haben sich die folgenden Gruppen als besonders geeignet erwiesen und werden in einer Menge von 0,01 bis 8,0 Gew.-% eingesetzt. Diese Menge wird auch nicht unter- oder überschritten, wenn eine Mischung unterschiedlicher Verbindungen der quartären Ammoniumverbindungen verwendet wird.

Erfindungsgemäß bevorzugte quartäre Ammoniumverbindungen sind ausgewählt aus mindestens einer der Gruppen
- der Esterquats und/oder
- der quaternierten polymeren Alkyloligoglucosid und/oder
- der quarternären Imidazoline der Formel (Tkat2), 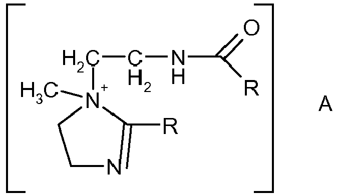 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
- der Amine und/oder kationisierten Amine und/oder
- Cetrimoniumchlorid und/oder Behentrimoniumchlorid, und/oder
- Poly(methacryloyloxyethyltrimethylammoniumverbindungen) und/oder
- quaternisierten Cellulose-Derivaten, insbesondere Polyquaternium 10 und/oder Polyquaternium-24 und/oder Polyquaternium-67 und/oder Polyquaternium-72 und/oder
- kationisiertem Honig und/oder
- kationischen Guar-Derivaten und/oder
- Chitosan und/oder
- polymeren Dimethyldiallylammoniumsalzen und deren Copolymeren mit Estern und Amiden von Acrylsäure und Methacrylsäure und/oder
- Copolymeren des Vinylpyrrolidons mit quaternierten Derivaten des Dialkylaminoalkylacrylats und -methacrylats und/oder
- Vinylpyrrolidon -Vinylimidazoliummethochlorid-Copolymeren und/oder
- quaterniertem Polyvinylalkohol.

Esterquats gemäß der Formel (Tkat1-1) bilden die erste Gruppe.

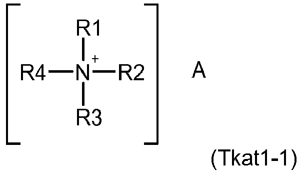

In der Formel (Tkat1) stehen R1, R2, R3 und R4 jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1-1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethyl-ammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat.

Esterquats gemäß der Formel (Tkat1-2) bilden eine bevorzugte Gruppe.

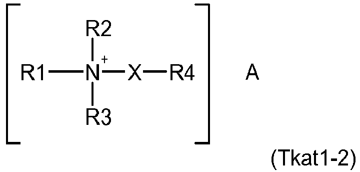

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- X - R4), mit der Maßgabe, dass höchstens 2 der Reste R1, R2 oder R3 für diesen Rest stehen können:
   Der Rest -(X - R4) ist mindestens 1 bis 3 mal enthalten.

Hierin steht X für:
1) -(CH2)n- mit n = 1 bis 20, vorzugsweise n = 1 bis 10 und besonders bevorzugt n = 1 - 5, oder
2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
3) eine Hydroxyalkylgruppe mit ein bis vier Kohlenstoffatomen, welche verzweigt oder unverzweigt sein kann, und welche mindestens eine und höchstens 3 Hydroxygruppen enthält. Beispiele sind: -CH₂OH, -CH₂CH₂OH, -CHOHCHOH, -CH₂CHOHCH₃, -CH(CH₂OH)₂, -COH(CH₂OH)₂, -CH₂CHOHCH₂OH, -CH₂CH₂CH₂OH und Hydroxybutylreste,
   und R4 steht für:
   1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
   2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,
   und A steht für ein physiologisch verträgliches organisches oder anorganisches Anion und wird an dieser Stelle stellvertretend für alle auch im Folgenden beschriebenen Strukturen definiert. Das Anion aller beschriebenen kationischen Verbindungen ist ausgewählt aus den Halogenidionen, Fluorid, Chlorid, Bromid, Iodid, Sulfaten der allgemeinen Formel RSO₃⁻, worin R die Bedeutung von gesättigtem oder ungesättigtem Alkylresten mit 1 bis 4 Kohlenstoffatomen hat, oder anionischen Reste organischer Säuren wie Maleat, Fumarat, Oxalat, Tartrat, Citrat, Lactat oder Acetat.

Solche Produkte werden beispielsweise unter den Warenzeichen Rewoquat^{®}, Stepantex^{®}, Dehyquart^{®}, Armocare^{®} und Akypoquat^{®} vertrieben. Die Produkte Armocare^{®} VGH-70, Dehyquart^{®} F-75, Dehyquart^{®} C-4046, Dehyquart^{®} L80, Dehyquart^{®} F-30, Dehyquart^{®} AU-35, Rewoquat^{®} WE18, Rewoquat^{®} WE38 DPG, Stepantex^{®} VS 90 und Akypoquat^{®} 131 sind Beispiele für diese Esterquats.

Weitere erfindungsgemäß besonders bevorzugte Verbindungen der Formel (Tkat1-2) zählen zur Formel (Tkat1-2.1), den kationischen Betainestern.

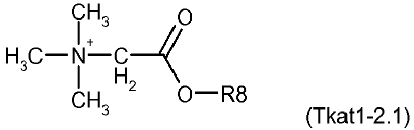

R8 entspricht in seiner Bedeutung R7.

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe erfindungsgemäß hervorragend zu verwendender quartären Ammoniumverbindungen sind Polymere auf der Basis von Glucose. Die folgende Abbildung zeigt ein derartiges kationisches Alkyloligoglucosid.

Ein Beispiel davon wird in der folgenden Abbildung gezeigt:

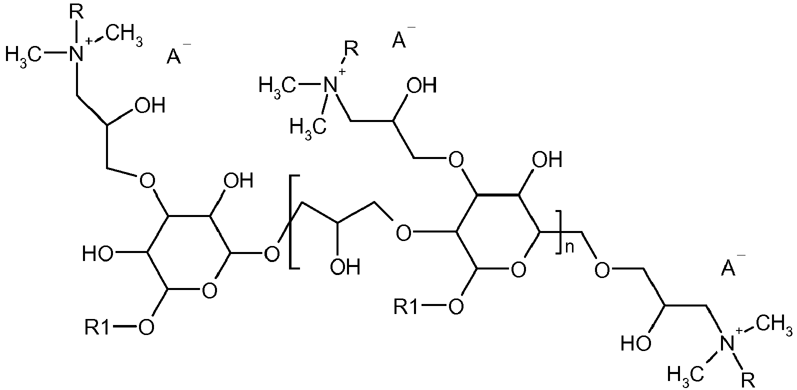

In der zuvor dargestellten Formel stehen die Reste R unabhängig voneinander für einen linearen oder verzweigten C6 bis C30 Alkylrest, einen linearen oder verzweigten C6 - C30 Alkenylrest, vorzugsweise steht der Rest R für einen Rest R ausgewählt aus: Lauryl, Myristyl, Cetyl, Stearyl, Oleyl, Behenyl oder Arachidyl, und A⁻ steht für ein physiologisch verträgliches Anion.

Die Reste R1 stehen unabhängig voneinander für einen linearen oder verzweigten C6 bis C30 Alkylrest, einen linearen oder verzweigten C6 bis C30 Alkenylrest, vorzugsweise steht der Rest R für einen Rest ausgewählt aus: Butyl, Capryl, Caprylyl, Octyl, Nonyl, Decanyl, Lauryl, Myristyl, Cetyl, Stearyl, Oleyl, Behenyl oder Arachidyl. Besonders bevorzugt sind die Reste R1 gleich. Noch bevorzugter sind die Reste R1 ausgewählt aus technischen Mischungen der Fettalkoholschnitte aus C6/C8 - Fettalkoholen, C8/C10 - Fettalkoholen, C10/C12 - Fettalkoholen, C12/C14 - Fettalkoholen, C12 / C18 - Fettalkoholen, und höchst bevorzugt sind hierbei diejenigen technischen Fettalkoholschnitte, welche pflanzlichen Ursprunges sind.

Die zuvor dargestellten kationischen Alkyloligoglucoside können beispielsweise aus üblichen Alkyloligoglucosiden hergestellt werden. Die Alkyloligoglucoside werden in diesem Falle nach üblichen Methoden zu quartären Ammoniumverbindungen umgesetzt. Bei den Alkyl- oder Alkenyloligoglykosiden kann es sich um bekannte nichtionische Tenside handeln. Diese Zuckertenside stellen bekannte nichtionische Tenside gemäß Formel (I) dar,

R¹O-[G]ₚ (I)

in der R¹ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht.

Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht für eine Zahl zwischen 1 und 30. Während p im einzelnen Molekül stets ganzzahlig sein muß und hier vor allem die Werte p = 1 bis 20 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 20,0 eingesetzt. Der Alkyl- bzw. Alkenylrest R¹ kann sich von primären Alkoholen mit 4 bis 30, vorzugsweise 6 bis 24 Kohlenstoffatomen, besonders bevorzugt 8 bis 22 Kohlenstoffatomen ableiten. Typische Beispiele sind Butanol, Capronalkohol, Caprylalkohol, Caprinalkohol, Octanol, Nonanol, Decanol, Undecylalkohol, Laurylalkohol, Myristylalkohol, Cetylalkohol, Oleylalkohol, Stearylalkohol, Behenylalkohol, Arachidylalkohol sowie technische Mischungen aus und mit diesen Alkoholen.

Die Quaternierung der Alkyloligoglukoside kann beispielsweise mit quarternären Ammoniumsalzen wie beispielsweise N,N-Dimethyl-N-(n-Alkyl)-N-(2-hydroxy-3-chloro-n-propyl)-ammoniumhalogen-iden durchgeführt werden. Die Kettenlänge der Alkylgruppe beträgt vorzugsweise 6 bis 30, bevorzugter 8 bis 24 Kohlenstoffatome.

Besonders bevorzugte Beispiele für die kationischen Alkyloligoglucoside sind die Verbindungen mit den INCI - Bezeichnungen Polyquaternium-77, Polyquaternium-78, Polyquaternium-79, Polyquaternium-80, Polyquaternium-81 und Polyquaternium-82. Höchst bevorzugt sind die kationischen Alkyloligoglucoside mit den Bezeichnungen Polyquaternium-77, Polyquaternium-81 und Polyquaternium-82.

Derartige Verbindungen können beispielsweise unter der Bezeichnung Poly Suga® Quat von der Fa. Colonial Chemical Inc. bezogen werden.

Erfindungsgemäß umfasst ist selbstverständlich auch, dass mehr Mischungen von kationischen Alkyloligoglucosiden verwendet werden können. Bevorzugt ist in diesem Falle, wenn jeweils ein langkettiges und ein kurzkettiges kationisches Alkyloligoglucosid gleichzeitig verwendet werden. Eine weitere Gruppe sind quartäre Imidazolinverbindungen. Die im Folgenden dargestellte Formel (Tkat2) zeigt die Struktur dieser Verbindungen.

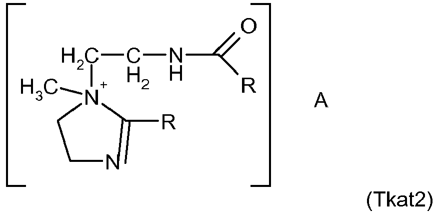

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCII - Bezeichnungen Quaternium-27, Quaternium-72, Quaternium-83 und Quaternium-91 erhältlich. Höchst bevorzugt ist erfindungsgemäß Quaternium-91.

In einer besonders bevorzugten Ausführungsform der Erfindung enthalten die erfindungsgemäßen Zusammensetzungen weiterhin mindestens ein Amin und/oder kationisiertes Amin, insbesondere ein Amidoamin und/oder ein kationisiertes Amidoamin mit den folgenden Strukturformeln:

R1 - NH - (CH₂)ₙ - N⁺R²R³R⁴ A (Tkat3)

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und
R2, R3 und R4 jeweils unabhängig voneinander
1) Wasserstoff oder
2) ein Alkylrest mit 1 bis 4 C-Atomen, welcher gleich oder verschieden, gesättigt oder ungesättigt sein kann, und
3) eine verzweigte oder unverzweigte Hydroxyalkylgruppe mit ein bis 4 Kohlenstoffatomen mit mindestens einer und höchstens drei Hydroxygruppen beispielsweise -CH₂OH, -CH₂CH₂OH, -CHOHCHOH, -CH₂CHOHCH₃, -CH(CH₂OH)₂, -COH(CH₂OH)₂, -CH₂CHOHCH₂OH, -CH₂CH₂CH₂OH und Hydroxybutylreste, und
   A ein Anion wie zuvor beschrieben und
   n eine ganze Zahl zwischen 1 und 10 bedeuten.

Bevorzugt wird eine Zusammensetzung, in welcher das Amin und/oder das quaternisierte Amin gemäß allgemeiner Formeln (Tkat3) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen. Als Beispiele hierfür kommen Lanolin, Bienen- oder Candellilawachse in Betracht.

Bevorzugt sind auch solche Amidoamine und/oder quaternisierte Amidoamine, in denen R2, R3 und/oder R4 in der Formel (Tkat3) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in der allgemeinen Formel (Tkat8) ist eine ganze Zahl zwischen 2 und 5.

Die Alkylamidoamine können sowohl als solche vorliegen und durch Protonierung in entsprechend saurer Lösung in eine quartäre Verbindung in der Zusammensetzung überführt werden. Erfindungsgemäß bevorzugt sind die kationischen Alkylamidoamine.

Beispiele für derartige erfindungsgemäße Handelsprodukte sind Witcamine^{®} 100, Incromine^{®} BB, Mackine^{®} 401und andere Mackine^{®} -Typen, Adogen^{®} S18V, und als permanent kationische Aminoamine: Rewoquat^{®} RTM 50, Empigen^{®} CSC, Swanol^{®} Lanoquat DES-50, Rewoquat^{®} UTM 50, Schercoquat^{®} BAS, Lexquat^{®} AMG-BEO, oder Incroquat^{®} Behenyl HE.

Weitere quartäre Ammoniumverbindungen sind kationische und amphotere Polymere.

Die kationischen und/oder amphoteren Polymere können Homo- oder Copolymere oder Polymere auf Basis natürlicher Polymere sein, wobei die quartären Stickstoffgruppen entweder in der Polymerkette oder vorzugsweise als Substituent an einem oder mehreren der Monomeren enthalten sind. Die Ammoniumgruppen enthaltenden Monomere können mit kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quartäre Vinylammoniummonomere mit cyclischen, kationische Stickstoffe enthaltenden Gruppen wie Pyridinium, Imidazolium oder quartäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl-und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Aus der Vielzahl dieser Polymere haben sich als besonders wirkungsvolle Bestandteile des erfindungsgemäßen Wirkstoffkomplexes erwiesen:

Homopolymere der allgemeinen Formel -{CH2-[CR1C00-(CH2)mN+R2R3R4]}n X-,

in der R1= -H oder -CH3 ist, R2, R3 und R4 unabhängig voneinander ausgewählt sind aus C1-4-Alkyl-, -Alkenyl- oder -Hydroxyalkylgruppen, m = 1, 2, 3 oder 4, n eine natürliche Zahl und

X- ein physiologisch verträgliches organisches oder anorganisches Anion ist. Im Rahmen dieser Polymere sind diejenigen erfindungsgemäß bevorzugt, für die mindestens eine der folgenden Bedingungen gilt: R1 steht für eine Methylgruppe, R2, R3 und R4 stehen für Methylgruppen, m hat den Wert 2.

Als physiologisch verträgliches Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfate und Halogenidionen, insbesondere Chlorid.

Ein besonders geeignetes Homopolymer ist das, gegebenenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare® CTH (Cosmetic Rheologies) und Synthalen® CR (3V Sigma) im Handel erhältlich. Das Homopolymer wird bevorzugt in Form einer nichtwässrigen Polymerdispersion eingesetzt. Solche Polymerdispersionen sind unter den Bezeichnungen Salcare® SC 95 und Salcare® SC 96 im Handel erhältlich.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel G-O-B-N+RₐR_{b}R_{c} A⁻
G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
Rₐ, R_{b} und R_{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in Rₐ, R_{b} und R_{c} vorzugsweise maximal 20 ist;
A⁻ ist ein übliches Gegenanion und ist vorzugsweise Chlorid.

Kationische, also quaternisierte Cellulosen sind mit unterschiedlichem Substitutionsgrad, kationischer Ladungsdichte, Stickstoffgehalt und Molekulargewichten auf dem Markt erhältlich. Beispielsweise wird Polyquaternium-67 im Handel unter den Bezeichnungen Polymer^{®} SL oder Polymer^{®} SK (Amerchol) angeboten. Unter der Handelsbezeichnung Mirustyle^{®} CP der Fa. Croda wird eine weitere höchst bevorzugte Cellulose angeboten. Diese ist eine Trimonium and Cocodimonium Hydroxyethylcellulose als derivatisierte Cellulose mit der INCI-Bezeichnung Polyquaternium-72. Polyquaternium-72 kann sowohl in fester Form als auch bereits in wässriger Lösung vorgelöst verwendet werden.

Weitere kationische Cellulosen sind Polymer JR^{®} 400 (Amerchol, INCI-Bezeichnung Polyquaternium-10) sowie Polymer Quatrisoft^{®} LM-200 (Amerchol, INCI-Bezeichnung Polyquaternium-24). Weitere Handelsprodukte sind die Verbindungen Celquat^{®} H 100 und Celquat^{®} L 200. Besonders bevorzugte kationische Cellulosen sind Polyquaternium-24, Polyquaternium-67 und Polyquaternium-72.

Geeignete kationische Guarderivate werden unter der Handelsbezeichnung Jaguar^{®} vertrieben und haben die INCI-Bezeichnung Guar Hydroxypropyltrimonium Chloride. Weiterhin sind besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia^{®} vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat. Die kationischen Guar-Derivate sind erfindungsgemäß bevorzugt.

Ein geeignetes Chitosan wird beispielsweise von der Firma Kyowa Oil& Fat, Japan, unter dem Handelsnamen Flonac^{®} vertrieben. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise
- kationische Alkylpolyglycoside,
- kationisierter Honig, beispielsweise das Handelsprodukt Honeyquat® 50,
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid -Acrylamid-Copolymer) im Handel erhältlichen Produkte sind Beispiele für solche kationischen Polymere mit der INCI - Bezeichnung Polyquaternium-7,
- Vinylpyrrolidon-Vinylimidazoliummethochlorid-Copolymere, wie sie unter den Bezeichnungen Luviquat® FC 370, FC 550 und der INCI-Bezeichnung Polyquaternium-16 sowie FC 905 und HM 552 angeboten werden,
- quaternisiertes Vinylpyrrolidon/Dimethylaminoethylmethacrylat, zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und die INCI - Bezeichnung Polyquaternium-11,
- quaternierter Polyvinylalkohol,
- sowie die unter den Bezeichnungen Polyquaternium-2, Polyquaternium-17, Polyquaternium 18 und Polyquaternium-27 bekannten Polymeren mit quartären Stickstoffatomen in der Polymerhau ptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex® SF 40 angeboten werden.

Erfindungsgemäße amphotere Polymere sind solche Polymerisate, in denen sich eine kationische Gruppe ableitet von mindestens einem der folgenden Monomere:
(i) Monomeren mit quartären Ammoniumgruppen der allgemeinen Formel (Mono1),

   R1-CH=CR2-CO-Z-(CnH2n)-N(+)R2R3R4 A(-) (Mono1)

   in der R1 und R2 unabhängig voneinander stehen für Wasserstoff oder eine Methylgruppe und R3, R4und R5 unabhängig voneinander für Alkylgruppen mit 1 bis 4 Kohlenstoff-Atomen, Z eine NH-Gruppe oder ein Sauerstoffatom, n eine ganze Zahl von 2 bis 5 und A(-) das Anion einer organischen oder anorganischen Säure ist,
(ii) Monomeren mit quartären Ammoniumgruppen der allgemeinen Formel (Mono2), 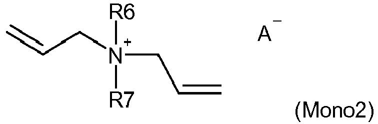 worin R6 und R7 unabhängig voneinander stehen für eine (C1 bis C4)-Alkylgruppe, insbesondere für eine Methylgruppe und
   A- das Anion einer organischen oder anorganischen Säure ist,
(iii) monomeren Carbonsäuren der allgemeinen Formel (Mono3),

   R8-CH=CR9-COOH (Mono3)

   in denen R8 und R9 unabhängig voneinander Wasserstoff oder Methylgruppen sind.

Besonders bevorzugt sind solche Polymerisate, bei denen Monomere des Typs (i) eingesetzt werden, bei denen R3, R4 und R5 Methylgruppen sind, Z eine NH-Gruppe und A(-) ein Halogenid-, Methoxysulfat- oder Ethoxysulfat-Ion ist; Acrylamidopropyl-trimethyl-ammoniumchlorid ist ein besonders bevorzugtes Monomeres (i). Als Monomeres (ii) für die genannten Polymerisate wird bevorzugt Acrylsäure verwendet.

Besonders bevorzugte amphotere Polymere sind Copolymere, aus mindestens einem Monomer (Mono1) bzw. (Mono2) mit dem Momomer (Mono3), insbesondere Copolymere aus den Monomeren (Mono2) und (Mono3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquaternium-22 unter anderem mit dem Handelsnamen Merquat® 280 (Nalco) vertrieben.

Darüber hinaus können die erfindungsgemäßen amphoteren Polymere neben einem Monomer (Mono1) oder (Mono2) und einem Monomer (Mono3) zusätzlich ein Monomer (Mono4)
(iv) monomere Carbonsäureamide der allgemeinen Formel (Mono4), 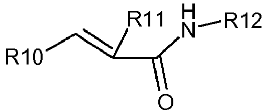 in denen R10 und R11 unabhängig voneinander Wasserstoff oder Methylgruppen sind und R12 für ein Wasserstoffatom oder eine (C1- bis C8)-Alkylgruppe steht, enthalten.

Erfindungsgemäß ganz besonders bevorzugt verwendete amphotere Polymere auf Basis eines Comonomers (Mono4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat® Plus 3330 (Nalco) vertrieben.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

Die zuvor genannten kationischen Polymere können einzeln oder in beliebigen Kombinationen miteinander verwendet werden.

Erfindungsgemäß bevorzugte Haarbehandlungsmittel sind dadurch gekennzeichnet, dass die quarternäre Ammoniumverbindung b) ausgewählt ist aus
i) den Esterquats und/oder
ii) den quaternierten polymeren Alkyloligoglucosiden und/oder
ii) den quarteren Imidazolinen der Formel (Tkat2), 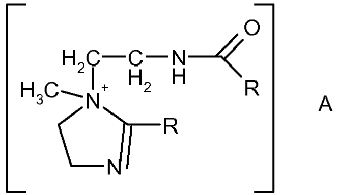 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht.
   oder beliebigen Mischungen hieraus.

Die quartären Ammoniumverbindungen b) sind in den erfindungsgemäßen Haarbehandlungsmitteln in Gewichtanteilen am Gesamtgewicht des Mittels zwischen 0,1 und 8 Gew.-%, vorzugsweise zwischen 0,2 und 5 Gew.-%, und insbesondere zwischen 0,3 und 3 Gew.- % erhalten.

Der dritte obligatorische Bestandteil des erfindungsgemäßen Wirkstoffkomplexes sind Esteröle. Erfindungsgemäß bevorzugte Esteröle weisen bei einem Druck von 1013 mbar einen Schmelzpunkt kleiner als 30 °C auf und besitzen mindestens eine (gegebenenfalls substituierte) Alkyloxycarbonyl-Gruppe im Molekül.

Bevorzugte erfindungsgemäße Haarbehandlungsmittel sind dadurch gekennzeichnet, dass der Gewichtanteil des Esteröls c) am Gesamtgewicht des Mittels zwischen 0,01 und 8 Gew.-%, vorzugsweise zwischen 0,05 und 5 Gew.-%, und insbesondere zwischen 0,2 und 2 Gew.-% beträgt.

Bevorzugte erfindungsgemäße Haarbehandlungsmittel sind solche, in denen mindestens ein Esteröl enthalten ist, das aus (C₄ bis C₂₀)-Monocarbonsäureestern von (C₂ bis C₂₀)-Alkoholen und/oder Dialkylcarbonaten und/oder Diestern von (C₂ bis C₂₀)-Alkoholen mit α- oder ω-(C₄ bis C₈)-Carbonsäuren und/oder Diestern von (C₂ bis C₆)-Diolen mit (C₃ bis C₁₀)-Monocarbonsäuren ausgewählt ist.

Erfindungsgemäß sind solche Mittel bevorzugt geeignet, in denen dass das Esteröl ausgewählt wird aus mindestens einer Verbindung der Formel (E-I) und/oder der Formel (E-II) und oder der Formel (E-III) 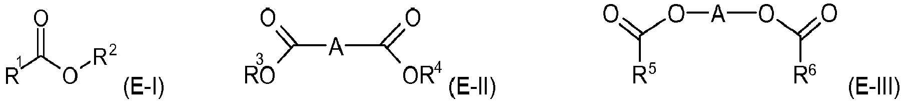 worin
- R¹: steht für einen linearen (C₂ bis C₂₀)-Kohlenwasserstoffrest, einen verzweigten (C₃ bis C₂₀)-Kohlenwasserstoffrest, eine (C₂ bis C₆)-Hydroxyalkylgruppe, eine lineare (C₂ bis C₁₈)-Alkoxygruppe oder eine verzweigte (C₃ bis C₁₈)-Alkoxygruppe,
- R²: steht für einen linearen (C₂ bis C₂₀)-Kohlenwasserstoffrest oder einen verzweigten (C₃ bis C₂₀)-Kohlenwasserstoffrest,
- R³ und R⁴: stehen unabhängig voneinander für eine lineare (C₂ bis C₁₀)-Alkylgruppe oder eine verzweigte (C₂ bis C₁₀)-Alkylgruppe,
- R⁵ und R⁶: stehen unabhängig voneinander für eine lineare (C₂ bis C₉)-Alkylgruppe oder eine verzweigte (C₃ bis C₁₀)-Alkylgruppe,
- A¹ und A²: stehen unabhängig voneinander für eine (C₂ bis C₈)-Alkandiylgruppe.

Beispiele für lineare (C₂ bis C₂₀)-Kohlenwasserstoffreste sind Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Oleyl.

Beispiele für verzweigte (C₃ bis C₂₀)-Kohlenwasserstoffreste sind Isopropyl, 2-Ethylhexyl, Heptan-3-yl, 2-Octyldodecyl, Isostearyl, Isocetyl.

Bevorzugte Reste R¹ gemäß Formel (E-I) sind 1-Hydroxyethyl, Heptyl, Heptan-3-yl, Octan-2-yl, Nonyl, Undecanyl, Tridecanyl, Pentadecanyl, Heptadecanyl.

Bevorzugte Reste R² gemäß Formel (E-I) sind Isopropyl, 2-Ethylhexyl, Octyl, Decyl, Dodecyl, 2-Octyldodecyl, Hexadecyl, Isohexadecyl (i.e. Isocetyl), Oleyl, Octadecyl oder Isooctadecyl (i.e. Isostearyl).

Bevorzugte Reste R³ und R⁴ gemäß Formel (E-II) sind unabhängig voneinander Isopropyl, n-Butyl, 2-Ethylhexyl oder n-Octyl.

Bevorzugte Reste A¹ gemäß Formel (E-II) stehen für Butan-1,4-diyl, Hexan-1,6-diyl oder Octan-1,8-diyl.

Bevorzugte Reste R⁵ und R⁶ gemäß Formel (E-III) sind unabhängig voneinander Heptyl, Heptan-3-yl, Octan-2-yl, Nonyl, Undecanyl, Tridecanyl, Pentadecanyl, Heptadecanyl..

Bevorzugte Reste A² gemäß Formel (E-III) stehen für Ethan-1,2-diyl, Propan-1,3-diyl, Butan-1,3-diyl, Butan-1,4-diyl oder Pentan-2,4-diyl.

Die erfindungsgemäßen Effekte sind besonders ausgeprägt, wenn das Mittel als Esteröl mindestens eine Verbindung ausgewählt aus Isopropylmyristat, 2,2-Dimethylpropan-1,3-diyl-di(2-ethylhexanoat) (CAS Nr.: 28510-23-8, z.B. Schercemol^{®} NGDO Ester der Firma Lubrizol) Diisopropyladipat, Di-n-butyladipat, Dioctylmaleat, Diisopropylsebacat, Tridecylneopentanoat (z.B. Ceraphyl^{®} 55 der Firma ISP), Decyloleat (z.B. Ceraphyl^{®} 140 der Firma ISP), Isostearylneopentanoat (z.B. Ceraphyl^{®} 375 der Firma ISP), Isocetylstearat (z.B. Ceraphyl^{®} 494 der Firma ISP), 2-Octyldodecylstearat (z.B. Ceraphyl^{®} ODS der Firma ISP), Isononansäure-C₁₆₋₁₈- alkylester, 2-Ethylhexylpalmitat, 2-Ethylhexylsäurecetylester (z.B. Schercemol^{®} CO Ester der Firma Lubrizol) Stearinsäure-2-ethylhexylester, Cetyloleat, Kokosfettalkohol-caprinat/-caprylat, n-Butylstearat, Oleylerucat, Isopropylpalmitat, Oleyl Oleate, Laurinsäurehexylester, Cetearyisononanoat, Lauryllactat (z.B. Ceraphyl^{®} 31 der Firma ISP), (C12 bis C15)-Alkyllactat (z.B. Ceraphyl^{®} 41 der Firma ISP), Dihexylcarbonat, Dioctylcarbonat (z.B. Cetiol^{®} CC der Firma Cognis), Didecylcarbonat enthalten ist.

Erfindungsgemäße Haarbehandlungsmittel enthalten vorzugsweise weiterhin mindestens einen natürlichen oder synthetischen kosmetischen Ölkörper, vorzugsweise ein Paraffinöl.

Geeignete natürliche oder synthetische kosmetische Ölkörper sind beispielweise:
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäureester von Polyhydroxyverbindungen. Unter Polyhydroxyverbindungen im Sinne der Erfindung werden alle Substanzen verstanden, welche die Definition in Römpp's Lexikon der Chemie, Version 2.0 der CD - ROM Ausgabe von 1999, Verlag Georg Thieme, erfüllen. Demnach sind unter Polyhydroxyverbindungen organische Verbindungen mit mindestens zwei Hydroxygruppen zu verstehen. Insbesondere sind im Sinne der vorliegenden Erfindung hierunter zu verstehen:
- Polyolfettsäureester, wie beispielsweise das Handelsprodukt Hydagen^{®} HSP (Cognis) oder Sovermol - Typen (Cognis), aus Fettsäure und/oder Fettsäurederivaten und Polyolen mit mindestens zwei Hydroxygruppen, und mit einer Kohlenstoffkette von 2 bis 30 Kohlenstoffatomen wie beispielsweise Fettsäureester von Trimethylolpropan,
- Fettsäureester von Kohlenhydraten, Zuckeralkoholen und Zuckern, insbesondere Fettsäureester von Monosacchariden, Disacchariden, Trisacchariden und Oligosacchariden, wobei diese auch in Form von Aldosen, Ketosen und/oder Lactosen sowie in Form der Methylether und als Phosphatester, vorliegen können.

Die erfindungsgemäßen Mittel können in verschiedenen Formen, beispielsweise als Lotion, Öl-in-Wasser-Emulsion oder Wasser-in-Öl-Emulsion, vorliegen.

Die erfindungsgemäßen Mittel enthalten die Wirkstoffe in einem kosmetischen Träger. Dieser kosmetische Träger ist im Sinne der Erfindung wässrig, alkoholisch oder wässrig-alkoholisch.

Ein wässriger Träger enthält im Sinne der Erfindung mindestens 20 Gew.-%, insbesondere mindestens 50 Gew.-% Wasser, bezogen auf das Gesamtgewicht des kosmetischen Mittels. Erfindungsgemäß bevorzugte Haarbehandlungsmittel enthalten, bezogen auf ihr Gesamtgewicht, 20 bis 98 Gew.-%, vorzugsweise 30 bis 95 Gew.-%, weiter bevorzugt 50 bis 95 Gew.-% und insbesondere 60 bis 95 Gew.-% Wasser.

Unter wässrig-alkoholischen Trägern sind im Sinne der vorliegenden Erfindung wasserhaltige Zusammensetzungen zu verstehen, welche, bezogen auf ihr Gesamtgewicht 1,0 bis 20 Gew.-% C₁-C₆-Alkohol, vorzugsweise 2,0 bis 10 Gew.-% C₁-C₆-Alkohol enthalten. Bevorzugte C₁-C₆-Alkohole sind Ethanol, Isopropanol, 1,2-Propandiol, Butanol, Isobutanol, tert.-Butanol, n-Pentanol, iso-Pentanole, n-Hexanol, iso-Hexanole, Glykol, Glycerin, 1,2-Pentandiol, 1,5-Pentandiol, 1,2-Hexandiol oder 1,6-Hexandiol. Erfindungsgemäß bevorzugte Haarbehandlungsmittel sind dadurch gekennzeichnet, dass sie, bezogen auf ihr Gesamtgewicht, 20 bis 98 Gew.-%, vorzugsweise 30 bis 95 Gew.-% und insbesondere 60 bis 95 Gew.-% Wasser und Alkohol(e) enthalten.

Weiterhin ist es erfindungsgemäß höchst bevorzugt, wenn in den erfindungsgemäßen Mitteln mindestens eine ausgewählte Aminosäure und/oder mindestens ein ausgewähltes Oligopeptid und/oder mindestens ein ausgewähltes kationisches Proteinhydrolysat enthalten sind.

In der vorliegenden Anmeldung wird unter dem Begriff Aminosäure auch eine Struktur verstanden, die nur eine permanente kationische Gruppe im Molekül enthält wie beispielsweise Cholin. Weiterhin werden unter diesem Begriff auch Substanzen wie Carnitin oder Taurin verstanden, da sie wie die Aminosäuren in biologischen Systemen natürlicherweise vorkommen und sich in vielen Fällen wie Aminosäuren verhalten.

Erfindungsgemäße Aminosäuren sind ausgewählt aus Alanin, Arginin, Asparagin, Asparaginsäure, Cystein, Cystin, Glutaminsäure, Glutamin, Glycin, Histidin, Hydroxylysin, Hydroxyprolin, Isoleucin, Leucin, Lysin, Methionin, Phenylalanin, Prolin, Serin, Threonin, Thyroxin, Tryptophan, Tyrosin, Valin, Betain, Ornithin, 1,1-Dimethyl-Prolin, Hercynin (Nα,Nα,Nα-Trimethyl-L-histidinium-betain), Ergothionein (Thionein, 2-Mercapto-Nα, Nα, Nα-trimethyl-L-histidinium-betain), Carnitin, Taurin und Cholin sowie deren Mischungen. Es können erfindungsgemäß alle Arten von Isomeren, wie beispielsweise Diastereomere, Enantiomere, cis - trans-Isomere, optische Isomere, Konformationsisomere und Racemate verwendet werden.

Mit besonderem Vorzug werden Alanin, Arginin, Asparagin, Glutaminsäure, Glutamin, Glycin, Histidin, Hydroxylysin, Hydroxyprolin, Isoleucin, Leucin, Lysin, Prolin, Serin, Betain, Ornithin, 1,1-Dimethyl-Prolin, Carnitin, Taurin, Cholin, Valin sowie deren Mischungen verwendet.

Ganz besonders bevorzugt werden Arginin, Glutamin, Glycin, Histidin, Lysin, Prolin, Serin, Betain, Carnitin, Taurin, Valin sowie deren Mischungen verwendet.

Höchst bevorzugt werden Arginin, Glutamin, Glycin, Histidin, Prolin, Lysin, Carnitin, Taurin, Valin sowie deren Mischungen verwendet.

Allerhöchst bevorzugt sind Arginin, Glutamin, Carnitin und Taurin sowie die Mischungen aus: Arginin und Taurin; Glutamin und Taurin; Glutamin und Carnitin; Arginin und Glutamin; Carnitin und Taurin, sowie die Mischungen aus: Arginin, Carnitin und Taurin; Glutamin, Carnitin und Taurin. Alternativ und/oder gemeinsam mit den zuvor beschriebenen Aminosäuren sind erfindungsgemäß ausgewählte Oligopeptide verwendbar. Oligopeptide im Sinne der vorliegenden Anmeldung sind durch Peptid-Bindungen Säureamid-artig verknüpfte Kondensationsprodukte von Aminosäuren, die mindestens 3 und maximal 25 Aminosäuren umfassen. In erfindungsgemäß bevorzugten Haarbehandlungsmitteln umfaßt das Oligopeptid 5 bis 15 Aminosäuren, vorzugsweise 6 bis 13 Aminosäuren, besonders bevorzugt 7 bis 12 Aminosäuren und insbesondere 8, 9 oder 10 Aminosäuren.

Je nachdem, ob weitere Aminosäuren an die Sequenz Glu-Glu-Glu gebunden sind und je nach Art dieser Aminosäuren kann die Molmasse des in den erfindungsgemäßen Mitteln enthaltenen Oligopeptids variieren. Erfindungsgemäß bevorzugte Haarbehandlungsmittel sind dadurch gekennzeichnet, dass das Oligopeptid eine Molmasse von 650 bis 3000 Da, vorzugsweise von 750 bis 2500 Da, besonders bevorzugt von 850 bis 2000 Da und insbesondere von 1000 bis 1600 Da aufweist.

Wie aus der bevorzugten Anzahl von Aminosäuren in den Oligopeptiden und dem bevorzugten Molmassenbereich zu ersehen ist, werden vorzugsweise Oligopeptide eingesetzt, die nicht allein aus den drei Glutaminsäuren bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind.

So ist es bevorzugt, wenn die in den erfindungsgemäßen Mitteln eingesetzten Oligopeptide kein Methionin enthalten. Weiter bevorzugt ist es, wenn die in den erfindungsgemäßen Mitteln eingesetzten Oligopeptide kein Cystein und/oder Cystin enthalten. Weiter bevorzugt ist es, wenn die in den erfindungsgemäßen Mitteln eingesetzten Oligopeptide keine Asparaginsäure und/oder Asparagin enthalten. Weiter bevorzugt ist es, wenn die in den erfindungsgemäßen Mitteln eingesetzten Oligopeptide kein Serin und/oder Threonin enthalten. Demgegenüber ist es bevorzugt, wenn die in den erfindungsgemäßen Mitteln eingesetzten Oligopeptide Tyrosin enthalten. Weiter bevorzugt ist es, wenn die in den erfindungsgemäßen Mitteln eingesetzten Oligopeptide Leucin enthalten. Weiter bevorzugt ist es, wenn die in den erfindungsgemäßen Mitteln eingesetzten Oligopeptide Isoleucin enthalten. Weiter bevorzugt ist es, wenn die in den erfindungsgemäßen Mitteln eingesetzten Oligopeptide Arginin enthalten. Weiter bevorzugt ist es, wenn die in den erfindungsgemäßen Mitteln eingesetzten Oligopeptide Valin enthalten.

Besonders bevorzugte Oligopeptide bzw. in den bevorzugten Oligopeptiden enthaltene Aminosäuresequenzen werden nachstehend beschrieben:

Ein besonders bevorzugtes Oligopeptid enthält zusätzlich Tyrosin, das vorzugsweise über seine Säurefunktion an die Glu-Glu-Glu-Sequenz gebunden ist. Erfindungsgemäß bevorzugte Haarbehandlungsmittel sind daher dadurch gekennzeichnet, dass das in ihnen enthaltene Oligopeptid mindestens eine Aminosäuresequenz Tyr-Glu-Glu-Glu aufweist, wobei die Amino-Gruppe frei oder protoniert und die Carboxy-Gruppen frei oder deprotoniert vorliegen können.

Ein weiteres besonders bevorzugtes Oligopeptid enthält zusätzlich Isoleucin, das vorzugsweise über seine Aminofunktion an die Glu-Glu-Glu-Sequenz gebunden ist. Erfindungsgemäß bevorzugte Haarbehandlungsmittel sind daher dadurch gekennzeichnet, dass das in ihnen enthaltene das Oligopeptid mindestens eine Aminosäuresequenz Glu-Glu-Glu-lle aufweist, wobei die Amino-Gruppe frei oder protoniert und die Carboxy-Gruppen frei oder deprotoniert vorliegen können. Oligopeptide, die beide vorgenannten Aminosäuren (Tyrosin und Isoleucin) aufweisen, sind erfindungsgemäß bevorzugt. Besonders bevorzugt sind dabei erfindungsgemäße Haarbehandlungsmittel, bei denen das in ihnen enthaltene Oligopeptid mindestens eine Aminosäuresequenz Tyr-Glu-Glu-Glu-Ile aufweist, wobei die Amino-Gruppe frei oder protoniert und die Carboxy-Gruppen frei oder deprotoniert vorliegen können.

Weiter bevorzugte Oligopeptide enthalten zusätzlich Arginin, das vorzugsweise an Isoleucin gebunden vorliegt. Ein höchst bevorzugtes Oligopeptid ist unter der Handelsbezeichnung ProSina® von der Fa. Croda im Handel erhältlich.

Eine weitere erfindungsgemäße Komponente, welche wie die ausgewählten Aminosäuren und/oder ausgewählten Oligopeptide verwendet werden können, sind ausgewählte kationische Proteinhydrolysate. Mit ganz besonderem Vorzug werden erfindungsgemäß kationische Keratinhydrolysate verwendet. Unter diesen ist ein kationisches Keratinhydrolysat bevorzugt, welches der Formel (K-I) entspricht,

R'-X-R" (K-I),

in der
- R' für einen geradkettigen oder verzweigten, gesättigten oder ungesätttigten Kohlenwasserstoffrest mit 11 bis 24 Kohlenstoffatomen steht,
- R" ein Protein, ein Peptid oder ein Proteinhydrolysat bedeutet,
- X für -C(O)O- oder -N⁺(R^{III}₂)R^{IV}- oder -N(R^{III})R^{IV}- oder -C(O)-N(R^{V})R^{VI} - steht,
- R^{III} -(CH₂)ₓ-CH₃ mit x = 0 - 22 bedeutet und
- R^{IV} -CH₂-CH(OH)-CH₂- oder -(CH₂)ₓ- mit x = 0 - 22 bedeutet;
- R^{V} und R^{VI} unabhängig voneinander für -H oder -(CH₂)ₓ-CH₃ mit x = 0 - 22 stehen; mit der Maßgabe, dass R" für Keratin oder ein Keratinhydrolysat steht.

Höchst bevorzugt steht der Rest R^{I} für eine Laurylgruppe und X für -N⁺R^{III}₂R^{IV}-, besonders bevorzugt mit R^{III} stehend für -CH₃ und mit R^{IV} stehend für -CH₂-CH(OH)-CH₂- und R" steht höchst bevorzugt für ein Hydrolysat gewonnen aus dem Kortex und/oder der Cuticula von keratinischen Fasern. Am bevorzugtesten ist das Produkt mit der INCI - Bezeichnung Laurdimonium Hydroxypropyl Hydrolyzed Keratin.

Die erfindungsgemäß bevorzugten Haarbehandlungsmittel sind dadurch gekennzeichnet, dass sie, bezogen auf ihr Gesamtgewicht, zusätzlich 0,01 bis 2,0 Gew.-%, vorzugsweise 0,01 bis 1 Gew.-%, insbesondere 0,05 bis 0,5 Gew.-% mindestens einer Verbindung d) aus der Gruppe der Aminosäuren, der Oligopeptide und der kationischen Proteinhydrolysate enthalten. Erfindungsgemäß bevorzugte Haarbehandlungsmittel sind dadurch gekennzeichnet, dass mindestens eine Aminosäure ausgewählt aus Alanin, Arginin, Asparagin, Asparaginsäure, Cystein, Cystin, Citrullin, Glutaminsäure, Glutamin, Glycin, Histidin, Hydroxylysin, Hydroxyprolin, Isoleucin, Leucin, Lysin, Methionin, Phenylalanin, Prolin, Serin, Threonin, Thyroxin, Tryptophan, Tyrosin, Acetyltyrosin, Valin, Betain, Ornithin, 1,1-Dimethyl-Prolin, Hercynin, Ergothionein und Taurin sowie deren Mischungen, und/oder mindestens ein kationisches Proteinhydrolysat ausgewählt aus den kationischen Keratinhydrolysaten enthalten ist.

Ein bevorzugter Gegenstand der vorliegenden Erfindung ist daher ein Haarbehandlungsmittel enthaltend in einem geeigneten kosmetischen Träger, jeweils bezogen auf sein Gesamtgewicht,
a) mindestens ein Homo- oder Copolymer in einer Gesamtmenge von 0,01 bis 10 Gew.-%, enthaltend mindestens ein Monomer aus der Gruppe der Pflanzenöle,
b) mindestens eine quartäre Ammoniumverbindung in einer Gesamtmenge von 0,01 bis 8 Gew.-%,
c) mindestens ein Esteröl in einer Gesamtmenge von 0,01 bis 10 Gew.-%,
d) mindestens eine Verbindung d) in einer Gesamtmenge von 0,01 bis 2 Gew.-%, ausgewählt aus der Gruppe der Aminosäuren, der Oligopeptide und der kationischen Proteinhyd rolysate.

Im Rahmen einer bevorzugten Ausführungsform enthalten die erfindungsgemäßen Mittel zusätzlich mindestens ein Tensid.

Bevorzugte Tenside werden unabhängig voneinander ausgewählt unter anionischen Tensiden, zwitterionischen Tensiden, amphoteren Tenisden, nichtionischen Tensiden, kationischen Tensiden. Es hat sich als besonders bevorzugt erwiesen, wenn die erfindungsgemäßen Mittel zusätzlich mindestens ein nichtionisches und/oder zwitterionisches Tensid und/oder amphoterens Tensid enthalten. In den erfindungsgemäßen Zusammensetzungen tragen diese Inhaltsstoffe möglicherweise erheblich zur Stabilisierung der Viskosität und des Lagerverhaltens bei. Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside,

Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (Tampho) werden solche oberflächenaktiven Verbindungen verstanden, die zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Besonders bevorzugte ampholytische Tenside sind das N-Kokosalkylaminopropionat, das Kokosacylaminoethylaminopropionat und das C₁₂ - C₁₈ - Acylsarcosin. Eine besonders bevorzugte Verbindung ist das Coco Betaine.

Erfindungsgemäß bevorzugte Haarbehandlungsmittel sind dadurch gekennzeichnet, dass sie, bezogen auf ihr Gesamtgewicht, weiterhin mindestens ein Tensid ausgewählt aus den nichtionischen und/oder zwitterionischen und/oder amphoterenTensiden in einer Gesamtmenge von 0,01 bis 5 Gew.-%, vorzugsweise von 0,05 bis 3 Gew.-%, insbesondere von 0,1 bis 2 Gew.-% enthalten.

Ein bevorzugter Gegenstand der vorliegenden Erfindung ist daher ein Haarbehandlungsmittel enthaltend in einem geeigneten kosmetischen Träger, jeweils bezogen auf sein Gesamtgewicht,
a) mindestens ein Homo- oder Copolymer enthaltend mindestens ein Monomer aus der Gruppe der Pflanzenöle in einer Gesamtmenge von 0,01 bis 10 Gew.-%,
b) mindestens eine quartäre Ammoniumverbindung in einer Gesamtmenge von 0,01 bis 8 Gew.-%,
c) mindestens ein Esteröl in einer Gesamtmenge von 0,01 bis 10 Gew.-%,
d) mindestens eine Verbindung d) ausgewählt aus der Gruppe der Aminosäuren, der Oligopeptide und der kationischen Proteinhydrolysate in einer Gesamtmenge von 0,01 bis 2 Gew.-%.
e) mindestens ein Tensid ausgewählt aus den nichtionischen und/oder zwitterionischen und/oder amphoteren Tensiden in einer Gesamtmenge von 0,01 bis 5 Gew.-%.

Ferner können die erfindungsgemäßen Mittel weitere Wirk-, Hilfs- und Zusatzstoffe enthalten, wie beispielsweise nichtionische Polymere, kationische Polymere, zwitterionische Polymere, anionische Polymere, Strukturanten wie Glucose, Maleinsäure und Milchsäure, haarkonditionierende Verbindungen wie Phospholipide, faserstrukturverbessernde Wirkstoffe, insbesondere Mono-, Di-und Oligosaccharide wie beispielsweise Glucose, Galactose, Fructose, Fruchtzucker und Lactose, Entschäumer wie Silikone, Farbstoffe zum Anfärben des Mittels, Antischuppenwirkstoffe, Wirkstoffe wie Aminosäuren, Oligopeptide und Proteinhydrolysate, Polyphenole und (Pseudo)Ceramide, Panthenol, Pantothensäure, Pantolacton, Allantoin, Pyrrolidinoncarbonsäuren und deren Salze sowie Bisabolol, Vitamine, Provitamine und Vitaminvorstufen, insbesondere solche der Gruppen A, B₃, B₅, B₆, C, E, F und H, Pflanzenextrakte, Fette und Wachse wie Bienenwachs, Montanwachs und Paraffine, Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Trübungsmittel wie Latex, Styrol/PVP- und Styrol/Acrylamid-Copolymere, Perlglanzmittel wie Ethylenglykolmono- und -distearat sowie PEG-3-distearat, Pigmente, Aufhellverstärker (wie Natriumpersulfat, Kaliumpersulfat oder Ammoniumpersulfat), Treibmittel wie Propan-Butan-Gemische, N₂O, Dimethylether, CO₂ und Luft und Antioxidantien.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Wie eingangs ausgeführt, eignen sich die erfindungsgemäßen Mittel zur Behandlung von Haaren, insbesondere zur Pflege der Haare. Ein weiterer Erfindungsgegenstand ist daher ein Verfahren zur Behandlung keratinischer Fasern, dadurch gekennzeichnet, dass ein erfindungsgemäßes Haarbehandlungsmittel auf die keratinischen Fasern aufgetragen wird und dort bis zur nächsten Haarwäsche verbleibt ohne ausgespült zu werden.

In einer alternativen Variante erfindungsgemäßer Verfahren wird das erfindungsgemäße Haarbehandlungsmittel nach einer ausreichenden Einwirkzeit von den Haaren gespült. Ein weiterer Erfindungsgegenstand ist daher ein Verfahren zur Behandlung keratinischer Fasern, dadurch gekennzeichnet, dass ein erfindungsgemäßes Haarbehandlungsmittel auf die keratinischen Fasern aufgetragen und nach einer Einwirkzeit von 5 Sekunden bis 5 Minuten wieder ausgespült wird.

Bezüglich weiterer bevorzugter Ausführungsformen der erfindungsgemäßen Verfahren gilt mutatis mutandis das zu den erfindungsgemäßen Mitteln Gesagte.

Die erfindungsgemäßen Mittel eignen sich insbesondere zur Behandlung von Haaren, insbesondere zur Pflege der Haare, zur Verbesserung der Nass- und Trockenkämmbarkeit und zur Verbesserung der Widerstandsfähigkeit der Haaroberfläche gegenüber physikalischen Schädigungen, wie z.B. Kämmen und Föhnen, und ganz besonders bevorzugt zur Verbesserung des Anti-Spliss Effekt bzw. zur Verbesserung des Anti-Haarbruches. Ein weiterer Erfindungsgegenstand ist daher die Verwendung eines erfindungsgemäßen Haarbehandlungsmittels zur Behandlung von Haaren, insbesondere
- zur Pflege der Haaren
- zur Verbesserung der Nass- und Trockenkämmbarkeit
- zur Verbesserung der Widerstandsfähigkeit der Haaroberfläche gegenüber physikalischen Schädigungen, wie z.B. Kämmen und Föhnen,
und ganz besonders bevorzugt zur Verbesserung des Anti-Spliss Effekt bzw. zur Verbesserung des Anti-Haarbruches.

Bezüglich weiterer bevorzugter Ausführungsformen der erfindungsgemäßen Verwendungen gilt mutatis mutandis das zu den erfindungsgemäßen Mitteln Gesagte.

Die Zusammensetzung einiger bevorzugter kosmetischer Mittel kann den folgenden Tabellen entnommen werden (Angaben in Gew.-% bezogen auf das Gesamtgewicht des Haarbehandlungsmittels sofern nicht anders angegeben).

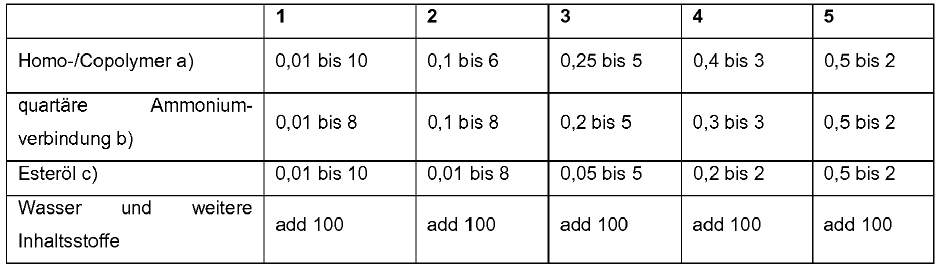

| | **6** | **7** | **8** | **9** | **10** |
|---|---|---|---|---|---|
| Brassica Campestris/ Aleurites Fordii Oil Copolymer | 0,01 bis 10 | 0,1 bis 6 | 0,25 bis 5 | 0,4 bis 3 | 0,5 bis 2 |
| Esterquat | 0,01 bis 8 | 0,1 bis 8 | 0,2 bis 5 | 0,3 bis 3 | 0,5 bis 2 |
| Esteröl c1 )* | 0,01 bis 10 | 0,01 bis 8 | 0,05 bis 5 | 0,2 bis 2 | 0,5 bis 2 |
| Wasser und weitere Inhaltsstoffe | add 100 | add 100 | add 100 | add 100 | add 100 |

| | | | | | |
|---|---|---|---|---|---|
| * Esteröl c1) entspricht in dieser wie in jeder der nachfolgenden Tabellen Verbindungen der Formel E-I | | | | | |

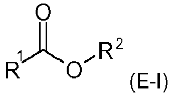

worin
- R¹: steht für einen linearen (C₂ bis C₂₀)-Kohlenwasserstoffrest, einen verzweigten (C₃ bis C₂₀)-Kohlenwasserstoffrest, eine (C₂ bis C₆)-Hydroxyalkylgruppe, eine lineare (C₂ bis C₁₈)-Alkoxygruppe oder eine verzweigte (C₃ bis C₁₈)-Alkoxygruppe,
- R²: steht für einen linearen (C₂ bis C₂₀)-Kohlenwasserstoffrest oder einen verzweigten (C₃ bis C₂₀)-Kohlenwasserstoffrest.

| | **11** | **12** | **13** | **14** | **15** |
|---|---|---|---|---|---|
| Homo-/Copolymer a) | 0,01 bis 10 | 0,1 bis 6 | 0,25 bis 5 | 0,4 bis 3 | 0,5 bis 2 |
| quartäre Ammonium-verbindung b) | 0,01 bis 8 | 0,1 bis 8 | 0,2 bis 5 | 0,3 bis 3 | 0,5 bis 2 |
| Esteröl c) | 0,01 bis 10 | 0,01 bis 8 | 0,05 bis 5 | 0,2 bis 2 | 0,5 bis 2 |
| Verbindung d) ** | 0,01 bis 2 | 0,01 bis 1,5 | 0,01 bis 1 | 0,05 bis 0,8 | 0,05 bis 0,5 |
| Wasser und weitere Inhaltsstoffe | add 100 | add 100 | add 100 | add 100 | add 100 |

| | | | | | |
|---|---|---|---|---|---|
| ** Verbindung d) entspricht in dieser wie in jeder der nachfolgenden Tabellen mindestens einer Verbindung aus der Gruppe der Aminosäuren, der Oligopeptide und der kationischen Proteinhydrolysate. | | | | | |

| | **16** | 1**7** | **18** | **19** | **20** |
|---|---|---|---|---|---|
| Brassica Campestris/ Aleurites Fordii Oil Copolymer | 0,01 bis 10 | 0,1 bis 6 | 0,25 bis 5 | 0,4 bis 3 | 0,5 bis 2 |
| Esterquat | 0,01 bis 8 | 0,1 bis 8 | 0,2 bis 5 | 0,3 bis 3 | 0,5 bis 2 |
| Esteröl c1 )* | 0,01 bis 10 | 0,01 bis 8 | 0,05 bis 5 | 0,2 bis 2 | 0,5 bis 2 |
| Hydrolysiertes Keratin | 0,01 bis 2 | 0,01 bis 1,5 | 0,01 bis 1 | 0,05 bis 0,8 | 0,05 bis 0,5 |
| Wasser und weitere Inhaltsstoffe | add 100 | add 100 | add 100 | add 100 | add 100 |

| | **21** | **22** | **23** | **24** | **25** |
|---|---|---|---|---|---|
| Homo-/Copolymer a) | 0,01 bis 10 | 0,1 bis 6 | 0,25 bis 5 | 0,4 bis 3 | 0,5 bis 2 |
| quartäre Ammonium-verbindung b) | 0,01 bis 8 | 0,1 bis 8 | 0,2 bis 5 | 0,3 bis 3 | 0,5 bis 2 |
| Esteröl c) | 0,01 bis 10 | 0,01 bis 8 | 0,05 bis 5 | 0,2 bis 2 | 0,5 bis 2 |
| Verbindung d) ** | 0,01 bis 2 | 0,01 bis 1,5 | 0,01 bis 1 | 0,05 bis 0,8 | 0,05 bis 0,5 |
| Tensid / Emulgatoren | 0,01 bis 5 | 0,05 bis 4 | 0,05 bis 3 | 0,1 bis 2 | 0,5 bis 2 |
| Wasser und weitere Inhaltsstoffe | add 100 | add 100 | add 100 | add 100 | add 100 |

| | **26** | **27** | **28** | **29** | **30** |
|---|---|---|---|---|---|
| Brassica Campestris/ Aleurites Fordii Oil Copolymer | 0,01 bis 10 | 0,1 bis 6 | 0,25 bis 5 | 0,4 bis 3 | 0,5 bis 2 |
| Esterquat | 0,01 bis 8 | 0,1 bis 8 | 0,2 bis 5 | 0,3 bis 3 | 0,5 bis 2 |
| Esteröl c1 )* | 0,01 bis 10 | 0,01 bis 8 | 0,05 bis 5 | 0,2 bis 2 | 0,5 bis 2 |
| Hydrolysiertes Keratin | 0,01 bis 2 | 0,01 bis 1,5 | 0,01 bis 1 | 0,05 bis 0,8 | 0,05 bis 0,5 |
| Tensid / Emulgatoren | 0,01 bis 5 | 0,05 bis 4 | 0,05 bis 3 | 0,1 bis 2 | 0,5 bis 2 |
| Wasser und weitere Inhaltsstoffe | add 100 | add 100 | add 100 | add 100 | add 100 |

| | **31** | **32** | **33** | **34** | **35** |
|---|---|---|---|---|---|
| Brassica Campestris/ Aleurites Fordii Oil Copolymer | 0,01 bis 10 | 0,1 bis 6 | 0,25 bis 5 | 0,4 bis 3 | 0,5 bis 2 |
| Esterquat | 0,01 bis 8 | 0,1 bis 8 | 0,2 bis 5 | 0,3 bis 3 | 0,5 bis 2 |
| Esteröl c1 )* | 0,01 bis 10 | 0,01 bis 8 | 0,05 bis 5 | 0,2 bis 2 | 0,5 bis 2 |
| Hydrolysiertes Keratin | 0,01 bis 2 | 0,01 bis 1,5 | 0,01 bis 1 | 0,05 bis 0,8 | 0,05 bis 0,5 |
| Konservierungsmittel | 0,01 bis 5 | 0,05 bis 4 | 0,1 bis 3 | 0,2 bis 2 | 0,2 bis 1 |
| Tensid / Emulgatoren | 0,01 bis 5 | 0,05 bis 4 | 0,05 bis 3 | 0,1 bis 2 | 0,5 bis 2 |
| Wasser und weitere Inhaltsstoffe | add 100 | add 100 | add 100 | add 100 | add 100 |

| | **36** | **37** | **38** | **39** | **40** |
|---|---|---|---|---|---|
| Brassica Campestris/ Aleurites Fordii Oil Copolymer | 0,01 bis 10 | 0,1 bis 6 | 0,25 bis 5 | 0,4 bis 3 | 0,5 bis 2 |
| Esterquat | 0,01 bis 8 | 0,1 bis 8 | 0,2 bis 5 | 0,3 bis 3 | 0,5 bis 2 |
| Esteröl c1 )* | 0,01 bis 10 | 0,01 bis 8 | 0,05 bis 5 | 0,2 bis 2 | 0,5 bis 2 |
| Hydrolysiertes Keratin | 0,01 bis 2 | 0,01 bis 1,5 | 0,01 bis 1 | 0,05 bis 0,8 | 0,05 bis 0,5 |
| Konservierungsmittel | 0,01 bis 5 | 0,05 bis 4 | 0,1 bis 3 | 0,2 bis 2 | 0,2 bis 1 |
| Tensid / Emulgatoren | 0,01 bis 5 | 0,05 bis 4 | 0,05 bis 3 | 0,1 bis 2 | 0,5 bis 2 |
| Wasser und weitere Inhaltsstoffe | add 100 | add 100 | add 100 | add 100 | add 100 |

| | **41** | **42** | **43** | **44** | **45** |
|---|---|---|---|---|---|
| Brassica Campestris/ Aleurites Fordii Oil Copolymer | 0,01 bis 10 | 0,1 bis 6 | 0,25 bis 5 | 0,4 bis 3 | 0,5 bis 2 |
| Esterquat | 0,01 bis 8 | 0,1 bis 8 | 0,2 bis 5 | 0,3 bis 3 | 0,5 bis 2 |
| Esteröl c1 )* | 0,01 bis 5 | 0,01 bis 4 | 0,05 bis 3 | 0,1 bis 1 | 0,5 bis 1 |
| Paraffinöl | 0,01 bis 5 | 0,01 bis 4 | 0,05 bis 3 | 0,1 bis 1 | 0,5 bis 1 |
| Hydrolysiertes Keratin | 0,01 bis 2 | 0,01 bis 1,5 | 0,01 bis 1 | 0,05 bis 0,8 | 0,05 bis 0,5 |
| Konservierungsmittel | 0,01 bis 5 | 0,05 bis 4 | 0,1 bis 3 | 0,2 bis 2 | 0,2 bis 1 |
| Tensid / Emulgatoren | 0,01 bis 5 | 0,05 bis 4 | 0,05 bis 3 | 0,1 bis 2 | 0,5 bis 2 |
| Wasser und weitere Inhaltsstoffe | add 100 | add 100 | add 100 | add 100 | add 100 |

## Patentansprüche

1. Haarbehandlungsmittel enthaltend in einem geeigneten kosmetischen Träger, jeweils bezogen auf sein Gesamtgewicht,
a) mindestens ein Homo- oder Copolymer enthaltend mindestens ein Monomer aus der Gruppe der Pflanzenöle in einer Gesamtmenge von 0,01 bis 10 Gew.-%,
b) mindestens eine quartäre Ammoniumverbindung in einer Gesamtmenge von 0,01 bis 8 Gew.-%,
c) mindestens ein Esteröl in einer Gesamtmenge von 0,01 bis 10 Gew.-%.

2. Haarbehandlungsmittel nach Anspruch 1, **dadurch gekennzeichnet, dass** es als Polymer a) ein unter der INCI-Bezeichnung: Brassica Campestris/Aleurites Fordii Oil Copolymer bekanntes Polymer enthält.

3. Haarbehandlungsmittel nach einem der vorherigen Ansprüche, **dadurch gekennzeichnet, dass** der Gewichtanteil der Polymer(e) a) am Gesamtgewicht des Mittels zwischen 0,1 und 10 Gew.-%, vorzugsweise zwischen 0,2 und 5 Gew.-%, und insbesondere zwischen 0,4 und 3 Gew.-% beträgt.

4. Haarbehandlungsmittel nach einem der vorherigen Ansprüche, **dadurch gekennzeichnet, dass** die quarternäre Ammoniumverbindung b) ausgewählt ist aus
i) den Esterquats und/oder
ii) den quaternierten polymeren Alkyloligoglucosiden und/oder
ii) den quarternären Imidazolinen der Formel (Tkat2), 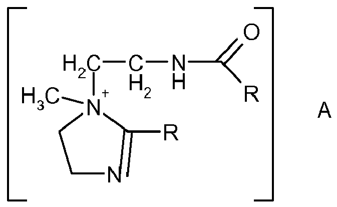 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht.
oder beliebigen Mischungen hieraus.

5. Haarbehandlungsmittel nach einem der vorherigen Ansprüche, **dadurch gekennzeichnet, dass** der Gewichtanteil der quarternären Ammoniumverbindung b) am Gesamtgewicht des Mittels zwischen 0,1 und 8 Gew.-%, vorzugsweise zwischen 0,2 und 5 Gew.-%, und insbesondere zwischen 0,3 und 3 Gew.-% beträgt.

6. Haarbehandlungsmittel nach einem der vorherigen Ansprüche, **dadurch gekennzeichnet, dass** das Esteröl c) aus (C₄ bis C₂₀)-Monocarbonsäureestern von (C₂ bis C₂₀)-Alkoholen und/oder Dialkylcarbonaten und/oder Diestern von (C₂ bis C₂₀)-Alkoholen mit α- oder ω-(C₄ bis C₈)-Carbonsäuren und/oder Diestern von (C₂ bis C₆)-Diolen mit (C₃ bis C₁₀)-Monocarbonsäuren ausgewählt ist.

7. Haarbehandlungsmittel nach einem der vorherigen Ansprüche, **dadurch gekennzeichnet, dass** der Gewichtanteil des Esteröls c) am Gesamtgewicht des Mittels zwischen 0,01 und 8 Gew.-%, vorzugsweise zwischen 0,05 und 5 Gew.-%, und insbesondere zwischen 0,2 und 2 Gew.-% beträgt.

8. Haarbehandlungsmittel nach einem der vorherigen Ansprüche, **dadurch gekennzeichnet, dass** es weiterhin mindestens ein natürlicher oder synthetischer kosmetische Ölkörper, vorzugsweise ein Paraffinöl enthält.

9. Haarbehandlungsmittel nach einem der vorherigen Ansprüche, **dadurch gekennzeichnet, dass** es, bezogen auf sein Gesamtgewicht, weiterhin mindestens ein Tensid ausgewählt aus den nichtionischen und/oder zwitterionischen und/oder amphoteren Tensiden in einer Gesamtmenge von 0,01 bis 5 Gew.-%, vorzugsweise von 0,05 bis 3 Gew.-%, insbesondere von 0,1 bis 2 Gew.-% enthält.

10. Haarbehandlungsmittel nach einem der vorherigen Ansprüche, **dadurch gekennzeichnet, dass** es, bezogen auf sein Gesamtgewicht, zusätzlich 0,01 bis 2,0 Gew.-%, vorzugsweise 0,01 bis 1 Gew.-%, insbesondere 0,05 bis 0,5 Gew.-% mindestens einer Verbindung d) aus der Gruppe der Aminosäuren, der Oligopeptide und der kationischen Proteinhydrolysate enthält.

11. Haarbehandlungsmittel nach Anspruch 10, **dadurch gekennzeichnet, dass** mindestens eine Aminosäure ausgewählt aus Alanin, Arginin, Asparagin, Asparaginsäure, Cystein, Cystin, Citrullin, Glutaminsäure, Glutamin, Glycin, Histidin, Hydroxylysin, Hydroxyprolin, Isoleucin, Leucin, Lysin, Methionin, Phenylalanin, Prolin, Serin, Threonin, Thyroxin, Tryptophan, Tyrosin, Acetyltyrosin, Valin, Betain, Ornithin, 1,1-Dimethyl-Prolin, Hercynin, Ergothionein und Taurin sowie deren Mischungen, und/oder mindestens ein kationisches Proteinhydrolysat ausgewählt aus den kationischen Keratinhydrolysaten enthalten sind.

12. Haarbehandlungsmittel nach einem der vorherigen Ansprüche, **dadurch gekennzeichnet, dass** es bezogen auf sein Gesamtgewicht, 20 bis 98 Gew.-%, vorzugsweise 30 bis 95 Gew.-%, weiter bevorzugt 50 bis 95 Gew.-% und insbesondere 60 bis 95 Gew.-% Wasser enthält.

13. Verfahren zur Behandlung keratinischer Fasern **dadurch gekennzeichnet, dass** ein Haarbehandlungsmittel gemäß einem der Ansprüche 1 bis 12 auf die keratinischen Fasern aufgetragen wird und dort bis zur nächsten Haarwäsche verbleibt ohne ausgespült zu werden.

14. Verfahren zur Behandlung keratinischer Fasern **dadurch gekennzeichnet, dass** ein Haarbehandlungsmittel gemäß einem der Ansprüche 1 bis 12 auf die keratinischen Fasern aufgetragen und nach einer Einwirkzeit von 5 Sekunden bis 5 Minuten wieder ausgespült wird.

15. Verwendung eines Haarbehandlungsmittels gemäß einem der Ansprüche 1 bis 12 zur Behandlung von Haaren, insbesondere
- zur Pflege der Haaren
- zur Verbesserung der Nass- und Trockenkämmbarkeit
- zur Verbesserung der Widerstandsfähigkeit der Haaroberfläche gegenüber physikalischen Schädigungen, wie z.B. Kämmen und Föhnen,
und ganz besonders bevorzugt zur Verbesserung des Anti-Spliss Effekt bzw. zur Verbesserung des Anti-Haarbruches.
